# EUROPÄISCHE PATENTSCHRIFT

(11) **EP 1 763 497 B1**
(45) Veröffentlichungstag und Bekanntmachung des Hinweises auf die Patenterteilung: **15.04.2009**
(21) Anmeldenummer: 05747587.3
(22) Anmeldetag: 04.06.2005
(51) Int. Cl.: C07C 1/24, C07C 15/46, C07C 11/02, C07C 13/11

(54) **VERFAHREN ZUR HERSTELLUNG VON ALKENEN DURCH ELIMINIERUNG VON WASSER AUS ALKOHOLEN MIT ALKYLPHOSPHONSÄUREANHYDRIDEN**
METHOD FOR PRODUCING ALKENES BY THE ELIMINATION OF WATER FROM ALCOHOLS, USING ALKYLPHOSPHONIC ACID ANHYDRIDES
PROCEDE POUR PRODUIRE DES ALCENES PAR ELIMINATION D'EAU A PARTIR D'ALCOOLS AU MOYEN D'ANHYDRIDES D'ACIDE ALKYLPHOSPHONIQUE

(30) Priorität: 19.06.2004 DE 102004029811
(43) Veröffentlichungstag der Anmeldung: 21.03.2007
(73) Patentinhaber: Archimica GmbH, 65929 Frankfurt am Main (DE)
(72) Erfinder: MEUDT, Andreas, 65719 Hofheim (DE); SCHERER, Stefan, 64347 Griesheim (DE); BÖHM, Claudius, 60594 Frankfurt am Main (DE)
(74) Vertreter: Schweitzer, Klaus
(86) Internationale Anmeldenummer: PCT/EP2005/006016
(87) Internationale Veröffentlichungsnummer: WO 2005/123632

(56) Entgegenhaltungen:
- EP-A- 0 370 399
- WO-A-86/04577

## Beschreibung

Alkene sind bedeutsame und äußerst vielseitig verwendbare Zwischenprodukte in der organischen Synthese. Diese Verbindungsklasse zeigt eine hohe Reaktivität der C,C-Doppelbindung, wodurch zahlreiche Additionsreaktionen ermöglicht werden. Die Bedeutung in der modernen organischen Synthese wird nur durch Limitierungen der Zugänglichkeit dieser Verbindungsklassen eingeschränkt. Standardverfahren zur Herstellung von Alkenen sind Eliminierungen entsprechend funktionalisierter Alkane. Dabei kommen zahllose Methoden wie die Abspaltung von Halogenwasserstoff und Halogenen, die Pyrolyse von Estern, Ammonium-, Phosphonium- Sulfonium- und Schwefelverbindungen und die Abspaltung von Wasserstoff zum Einsatz. Außerdem können aliphatische Hydroxyverbindungen in der Dampfphase oder in flüssiger Phase dehydratisiert werden wie z.B. in WO-A-86/04577 offenbart wird.

In der modernen organischen Synthese nimmt die Bedeutung chemo-, regio- und stereoselektiver Reagenzien explosionsartig zu. Will man beispielsweise aus einem komplexen Molekül mit zahlreichen funktionellen Gruppen eine bestimmte Alkoholfunktionalität in ein Alken überführen, so scheiden zahlreiche der genannten Methoden aus Selektivitätsgründen aus.

Eine selektive und bevorzugte Methode zur Eliminierung von Alkoholen zu Alkenen ist die Reaktion in flüssiger Phase. Die zur Überführung von Alkoholen in Olefine vorwiegend zur Anwendung kommenden Reagenzien lassen sich in alkalische und saure Dehydratisierungsmittel unterteilen. Daneben finden noch vereinzelt andere Verwendung, welche sich nicht streng einer dieser Gruppen zuordnen lassen.

Eine hochselektive Problemlösung für die genannten Transformationen fehlte bisher. Die bekannten Reagenzien können zwar die gewünschten Transformationen bewerkstelligen, dabei werden aber oft andere Gruppierungen ebenfalls beeinflusst. In vielen Fällen werden durch die erforderlichen drastischen Bedingungen selbst entfernt stehende Stereozentren epimerisiert.

Aufgabe ist es daher ein wirtschaftliches Verfahren zur Verfügung zu stellen, dass es erlaubt Alkohole durch Eliminierung von Wasser in die entsprechenden Alkene bei sehr milden Bedingungen zu überführen, wobei die Abtrennung gegebenenfalls gebildeter Folgeprodukte der eingesetzten Ausgangsprodukte einfach durchführbar sein soll.

Die im Stand der Technik bereits bekannten Verfahren zur Herstellung von Alkenen weisen alle gravierenden Nachteile auf.
So besteht bei der Verwendung von Schwefelsäure zur Wassereliminierung neben der Verkohlung und der Gefahr der Verunreinigung des Produktes mit Schwefeldioxid auch die Gefahr der Bildung von Ethern.
Bei Verwendung von Phosphorsäure zur Dehydratisierung sind meist Temperaturen von bis zu 220°C notwendig.
Borsäure findet nur gelegentlich Verwendung. Im Falle der Verwendung primärer Alkohole sind bereits Temperaturen von bis zu 300°C notwendig. Man erhält daher Olefingemische mit uneinheitlicher Lage der Doppelbindung.

Überraschenderweise wurde gefunden, dass der Einsatz von cyclischen 2,4,6-substituierten 1,3,5,2,4,6-trioxatriphosphinan-2,4,6-trioxiden alle diese Probleme löst. Diese Eliminierungsmethode ermöglicht die hochselektive Überführung von Alkoholen in die entsprechenden Alkene, wobei gleichzeitig die gewünschte Epimerisierungsfreiheit und maximale Regio- und Stereoselektivität bei zugleich nahezu quantitativen Ausbeuten beobachtet wird.

Die vorliegende Erfindung betrifft somit ein hochselektives Verfahren zur Herstellung von Alkenen der Formel (II)

R¹R²C = CR³R⁴ (II)

durch Umsetzung
a) primärer Alkohole (R¹R²CHCH₂ - OH) oder
b) sekundärer Alkohole (R¹R²CHCHR³ - OH) oder
c) tertiärer Alkohole (R¹R²CH - CR³R⁴OH)
mit cyclischen Alkylphosphonsäureanhydriden und gegebenenfalls einer Aminbase NR₃⁵, bei einer Temperatur im Bereich von -100 bis + 120°C, wobei R und/oder R¹ und/oder R² und/oder R³ und/oder R⁴ für H, einen linearen oder verzweigten, substituierten oder unsubstituierten C₁-C₁₂-Alkylrest, einen C₃-C₁₀ Cycloalkyl-, Alkenyl- oder einen Aryl- oder Heteroarylrest steht.

In einer bevorzugten erfindungsgemäßen Ausführungsform ist das cyclische Alkylphosphonsäureanhydrid ein 2,4,6-substituiertes 1,3,5,2,4,6-trioxatriphosphinan-2,4,6-trioxid der Formel (I), 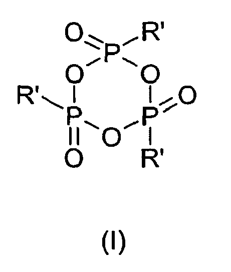 worin R' unabhängig voneinander für Allyl, Aryl oder offenkettige oder verzweigte C₁ bis C₁₂-Alkyl-Reste, insbesondere für C₁-C₈-Alkylreste steht.

Besonders bevorzugt werden Phosphonsäureanhydride der Formel (I) in denen R' für einen Methyl, Ethyl, n-Propyl, Isopropyl, n-Butyl, 2-Butyl, Isobutyl, Pentyl, Hexyl, insbesondere einen Ethyl-, Propyl- und/oder Butyl-Rest steht.

Die Eliminierung zu Alkenen (II) kann dabei im allgemeinen bei Temperaturen im Bereich von -100 bis +120°C durchgeführt werden, bevorzugt sind Temperaturen im Bereich von -30 bis +30°C, wobei tiefere Temperaturen im allgemeinen mit höheren Selektivitäten korreliert sind. Die Reaktionsdauer ist abhängig von der angewandten Temperatur und beträgt im allgemeinen 1 bis 12 Stunden, insbesondere 3 bis 6 Stunden.

Der Zusatz von Aminen ist im Allgemeinen nicht erforderlich, kann sich aber im Einzelfall als vorteilhaft erweisen. Als Amine werden im allgemeinen Amine der Formel (III)

NR⁵₃ (III)

eingesetzt, wobei R⁵ für H, Allyl, Aryl oder offenkettige, cyclische oder verzweigte C₁ bis C₁₂-Alkyl-Reste, Aryloxy, Allyloxy oder Alkoxy mit offenkettigen, cyclischen oder verzweigten C₁ bis C₁₂-Alkyl-Resten oder eine Kombination aus den genannten Substituenten steht.

Besonders bevorzugt werden Amine der Formel (III) in denen R⁵ für ein H, Methyl, Ethyl, n-Propyl, Isopropyl, n-Butyl, 2-Butyl, Isobutyl, Pentyl, Hexyl, Phenyl insbesondere ein H, Ethyl, n-Propyl, Isopropyl, n-Butyl, 2-Butyl, Isobutyl oder Phenyl oder eine Kombination aus den genannten Substituenten steht.

Das cyclische Phosphonsäureanhydrid kann dem Reaktionsmedium entweder als Schmelze oder als flüssige Mischung gelöst in einem Lösungsmittel zugegeben werden.
Geeignete Lösungsmittel sind dabei solche, die keine Nebenreaktionen mit dem Phosphonsäureanhydrid ergeben, dies sind alle aprotischen organischen Lösungsmittel, wie z.B. Ligroin, Butan, Pentan, Hexan, Heptan, Octan, Cyclobutan, Cyclopentan, Cyclohexan, Cycloheptan, Cyclooctan, Dichlormethan, Chloroform, Tetrachlorkohlenstoff, 1,2-Dichlorethan, 1,1,2,2-Tetrachlorethan, Methylacetat, Ethylacetat, Propylacetat, Butylacetat, Dimethylformamid, Diethylformamid, Dimethylacetamid, Diethylacetamid, Diethylether, Diisopropylether, tert-Butyl-Methylether, THF, Dioxan, Acetonitril oder Gemische aus diesen, besonders bevorzugt sind Dichlormethan, Chloroform, Ethylacetat, Propylacetat, Butylacetat, Dimethylformamid, Diethylformamid, Dimethylacetamid, Diethylacetamid, Diisopropylether, tert-Butyl-Methylether, THF, Dioxan, Acetonitril oder Gemische aus diesen, ganz besonders bevorzugt werden Dichlormethan, Chloroform, Ethylacetat, Butylacetat, Dimethylacetamid, tert-Butyl-Methylether, THF, Dioxan, Acetonitril oder Gemische aus diesen, insbesondere bevorzugt sind THF, Ethylacetat oder Butylacetat.

Die Zugabe des Phosphonsäureanhydrids erfolgt im allgemeinen mindestens drittelstöchiometrisch im Bezug auf die Ausgangsverbindung, kann aber auch überstöchiometrisch sein, beispielsweise im Verhältnis 1 Ausgangsverbindung : 1,2 T3P^{®} (cyclisches Propanphosphonsäureanhydrid).
Die Umsetzungen werden bevorzugt so durchgeführt, dass die entsprechende Ausgangsverbindung bei der Reaktionstemperatur zu T3P^{®} in einem geeigneten Lösungsmittel gegeben wird.

Die Isolierung des Reaktionsproduktes erfolgt bevorzugt durch Hydrolyse und einfache Phasentrennung, da die Folgeprodukte der Phosphonsäureanhydride allgemein sehr gut wasserlöslich sind. Je nach Natur des zu isolierenden Produkts können dabei auch Nachextraktionen erforderlich sein. Das gebildete Phosphonsäureanhydrid-Folgeprodukt stört Folgereaktionen oft nicht, so dass auch der direkte Einsatz der erhaltenen Reaktionslösungen oft sehr gute Ergebnisse bringt.
Alle benannten Verfahrensweisen zeichnen sich durch sehr gute Ausbeuten (typisch 90-100 %, insbesondere > 95 %) bei gleichzeitiger Abwesenheit von Nebenreaktionen und Epimerisierungen aus. Die Selektivitäten der erfindungsgemäßen Reaktion liegen im Bereich von 97-100 %, insbesondere 99-100 %.

Das erfindungsgemäße Verfahren soll durch die nachfolgenden Beispiele erläutert werden, ohne die Erfindung darauf zu beschränken:

### Beispiel 1: Eliminierung von 1-Phenylethanol zu Styrol

1 mol T3P^{®} in Ethylacetat (50 % w/w) wird auf 0°C gekühlt. Zu dieser Lösung wird 1-Phenylethanol getropft und 3 Stunden bei dieser Reaktion nachgerührt. Zu dieser Zeit zeigte das Reaktions-GC einen Umsatz von 100 % an. Nach Erwärmen auf Raumtemperatur wurden 180 ml Wasser zugeben und die Phasen getrennt. Nach Abkondensieren des Lösungsmittels verblieb das Styrol in einer Ausbeute von 97 %, HPLC-Reinheit 99 % (a/a).

### Beispiel 2: Eliminierung von 1-Octanol zu 1-Octen

1 mol T3P^{®} in Ethylacetat (50 % w/w) wird auf 0°C gekühlt. Zu dieser Lösung wird 1-Octanol getropft und 3 Stunden bei dieser Reaktion nachgerührt. Zu dieser Zeit zeigte das Reaktions-GC einen Umsatz von 100 % an. Nach Erwärmen auf Raumtemperatur wurden 180 ml Wasser zugeben und die Phasen getrennt. Nach Abkondensieren des Lösungsmittels verblieb das 1-Octen in einer Ausbeute von 97 %, GC-Reinheit 99 % (a/a).

### Beispiel 3: Eliminierung von 1-Cyclopentyl-prop-2-en-1-ol zu Allylidencyclopentan

1 mol T3P^{®} in Ethylacetat (50 % w/w) wird auf 0°C gekühlt. Zu dieser Lösung wird 1-Cyclopentyl-prop-2-en-1-ol getropft und 3 Stunden bei dieser Reaktion nachgerührt. Zu dieser Zeit zeigte das Reaktions-GC einen Umsatz von 100 % an. Nach Erwärmen auf Raumtemperatur wurden 180 ml Wasser zugeben und die Phasen getrennt. Nach Abkondensieren des Lösungsmittels verblieb das Allylidencyclopentan in einer Ausbeute von 97 %, GC-Reinheit 99 % (a/a).

### Beispiel 4: Eliminierung von (4R)-1-(tert-butoxycarbonyl)-4-hydroxy-L-prolin methylester zu 2,5-Dihydropyrrole-1,2-(2S)-dicarbonsäure 1-tert-butylester 2-methylester

1 mol T3P^{®} in Ethylacetat (50 % w/w) wird auf 0°C gekühlt. Zu dieser Lösung wird (4R)-1-(tert-butoxycarbonyl)-4-hydroxy-L-prolin methylester getropft und 3 Stunden bei dieser Reaktion nachgerührt. Zu dieser Zeit zeigte das Reaktions-GC einen Umsatz von 100 % an. Nach Erwärmen auf Raumtemperatur wurden 180 ml Wasser zugeben und die Phasen getrennt. Nach Abkondensieren des Lösungsmittels verblieb der 2,5-Dihydropyrrole-1,2-(2S)-dicarbonsäure 1-tert-butyl ester 2-methylester in einer Ausbeute von 97 %, GC-Reinheit 99 % (a/a).

## Patentansprüche

1. Verfahren zur Herstellung von a.) Alkenen der Formel (II)
R¹R²C = CR³R⁴ (II)
durch Umsetzung
a) primärer Alkohole R¹R²CHCH₂ - OH oder
b) sekundärer Alkohole R¹R²CHCHR³ - OH oder
c) tertiärer Alkohole R¹R²CH - CR³R⁴OH
mit cyclischen Alkylphosphonsäureanhydriden bei einer Temperatur im Bereich von -100 bis +120°C
wobei R und/oder R¹ und/oder R² und/oder R³ und/oder R⁴ für H, einen linearen oder verzweigten C₁-C₁₂-Alkylrest, einen C₃-C₁₀ Cycloalkyl-, Alkenyl- oder einen Aryl- oder Heteroarylrest steht.

2. Verfahren nach Anspruch 1, **dadurch gekennzeichnet, dass** das cyclische Alkylphosphonsäureanhydrid ein 2,4,6-substituiertes 1,3,5,2,4,6-trioxatriphosphinan-2,4,6-trioxid der Formel (I) ist, 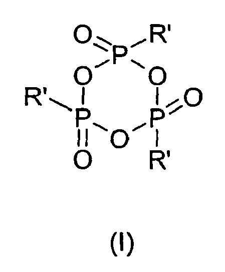 worin R' unabhängig voneinander für Allyl, Aryl oder offenkettige oder verzweigte C₁ bis C₁₂-Alkyl-Reste steht.

3. Verfahren nach Anspruch 2, **dadurch gekennzeichnet, dass** R' für einen Methyl, Ethyl, n-Propyl, Isopropyl, n-Butyl, 2-Butyl, Isobutyl, Pentyl; Hexyl, insbesondere einen Ethyl-, Propyl- und/oder Butyl-Rest steht.

4. Verfahren nach mindestens einem der vorhergehenden Ansprüche, **dadurch gekennzeichnet, dass** die Umsetzung in Gegenwart einer Aminbase der Formel (III) durchgeführt wird
NR⁵₃ (III)
eingesetzt, wobei R⁵ für H, Allyl, Aryl oder offenkettige, cyclische oder verzweigte C₁ bis C₁₂-Alkyl-Reste, Aryloxy, Allyloxy oder Alkoxy mit offenkettigen, cyclischen oder verzweigten C₁ bis C₁₂-Alkyl-Resten oder eine Kombination aus den genannten Substituenten steht.

5. Verfahren nach mindestens einem der vorhergehenden Ansprüche, **dadurch gekennzeichnet, dass** das cyclische Alkylphosphonsäureanhydrid entweder als Schmelze oder gelöst in einem Lösungsmittel der Reaktionslösung zugegeben wird.

6. Verfahren nach Anspruch 5, **dadurch gekennzeichnet, dass** das cyclische Alkylphosphonsäureanhydrid in einem aprotischen Lösungsmittel zugegeben wird.

7. Verfahren nach mindestens einem der vorhergehenden Ansprüche, **dadurch gekennzeichnet, dass** die Reaktionslösung vor Zugabe des Alkylphosphonsäureanhydrid auf die Reaktionstemperatur temperiert wird.

8. Verfahren nach mindestens einem der vorhergehenden Ansprüche, **dadurch gekennzeichnet, dass** das Alkylphosphonsäureanhydrid in Bezug auf die Ausgangsverbindung drittelstöchiometrisch bis überstöchiometrisch eingesetzt wird.

## Claims

1. A process for preparing a.) alkenes of the formula (II)
R¹R²C = CR³R⁴ (II)
by reacting
a) primary alcohols R¹R²CHCH₂-OH or
b) secondary alcohols R¹R²CHCHR³-OH or
c) tertiary alcohols R¹R²CH-CR³R⁴OH
with cyclic alkylphosphonic anhydrides at a temperature in the range from -100 to +120 °C,
where R and/or R¹ and/or R² and/or R³ and/or R⁴ is H, a linear or branched C₁-C₁₂-alkyl radical, a C₃-C₁₀-cycloalkyl radical, alkenyl radical or an aryl or heteroaryl radical.

2. The process as claimed in claim 1, wherein the cyclic alkylphosphonic anhydride is a 2,4,6-substituted 1,3,5,2,4,6-trioxatriphosphinane 2,4,6-trioxide of the formula (I) 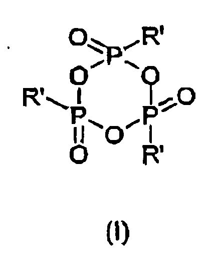 where R' is independently allyl, aryl or open-chain or branched C₁ to C₁₂-alkyl radicals.

3. The process as claimed in claim 2, wherein R' is a methyl, ethyl, n-propyl, isopropyl, n-butyl, 2-butyl, isobutyl, pentyl, hexyl, especially an ethyl, propyl and/or butyl radical.

4. The process as claimed in at least one of the preceding claims, wherein the reaction is performed in the presence of an amine base of the formula (III)
NR⁵₃ (III)
where R⁵ is H, allyl, aryl or open-chain, cyclic or branched C₁ to C₁₂-alkyl radicals, aryloxy, allyloxy or alkoxy having open-chain cyclic or branched C₁ to C₁₂-alkyl radicals, or a combination of the substituents mentioned.

5. The process as claimed in at least one of the preceding claims, wherein the cyclic alkylphosphonic anhydride is added to the reaction solution either as a melt or dissolved in a solvent.

6. The process as claimed in claim 5, wherein the cyclic alkylphosphonic anhydride is added in an aprotic solvent.

7. The process as claimed in at least one of the preceding claims, wherein the reaction solution is heated to the reaction temperature before the alkylphosphonic anhydride is added.

8. The process as claimed in at least one of the preceding claims, wherein the alkylphosphonic anhydride is used in one third of the stoichiometric amount up to a superstoichiometric amount in relation to the starting compound.

## Revendications

1. Procédé pour la préparation a.) d'alcènes de formule (II)
R¹R²C=CR³R⁴ (II)
par mise en réaction
a) d'alcools primaires R¹R²CHCH₂-OH ou
b) d'alcools secondaires R¹R²CHCHR³-OH ou
c) d'alcools tertiaires R¹R²CH-CR³R⁴OH
avec des anhydrides d'acide alkylphosphoniques cycliques, à une température dans la plage de -100 à + 120 °C
R et/ou R¹ et/ou R² et/ou R³ et/ou R⁴ représentant H, un radical alkyle en C₁-C₁₂ linéaire ou ramifié, un radical cycloalkyle en C₃-C₁₀, alcényle en C₃-C₁₀ ou un radical aryle ou hétéroaryle.

2. Procédé selon la revendication 1, **caractérisé en ce que** l'anhydride d'acide alkylphosphonique cyclique est un 1,3,5,2,4,6-trioxatriphosphinane-2,4,6-trioxyde 2,4,6-substitué de formule (I) 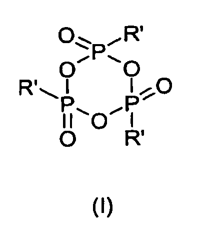 dans laquelle R' représentent, chacun indépendamment, des radicaux allyle, aryle ou alkyle en C₁-C₁₂ à chaîne droite ou ramifiée.

3. Procédé selon la revendication 2, **caractérisé en ce que** R' représente un radical méthyle, éthyle, n-propyle, isopropyle, n-butyle, 2-butyle, isobutyle, pentyle, hexyle, en particulier un radical éthyle, propyle et/ou butyle.

4. Procédé selon au moins l'une des revendications précédentes, **caractérisé en ce que** la réaction est effectuée en présence d'une base de type amine de formule (III)
NR⁵₃ (III)
R⁵ représentant H, un groupe allyle, aryle ou des radicaux alkyle en C₁-C₁₂ à chaîne droite, cycliques ou ramifiés, un groupe aryloxy, allyloxy ou alcoxy comportant des radicaux alkyle en C₁-C₁₂ à chaîne droite, cycliques ou ramifiés, ou une association des substituants cités.

5. Procédé selon au moins l'une des revendications précédentes, **caractérisé en ce qu'**on ajoute à la solution réactionnelle l'anhydride d'acide alkylphosphonique cyclique soit sous forme de masse fondue, soit dissous dans un solvant.

6. Procédé selon la revendication 5, **caractérisé en ce que** l'anhydride d'acide alkylphosphonique cyclique est ajouté dans un solvant aprotique.

7. Procédé selon au moins l'une des revendications précédentes, **caractérisé en ce que** la solution réactionnelle est portée à la température de réaction avant l'addition de l'anhydride d'acide alkylphosphonique.

8. Procédé selon au moins l'une des revendications précédentes, **caractérisé en ce que** l'anhydride d'acide alkylphosphonique est utilisé en une quantité allant du tiers à plus de la quantité stoechiométrique, par rapport au composé de départ.
